# EUROPEAN PATENT SPECIFICATION

(11) **EP 2 379 751 B1**
(45) Date of publication and mention of the grant of the patent: **20.03.2013**
(21) Application number: 10701404.5
(22) Date of filing: 13.01.2010
(51) Int. Cl.: C12Q 1/68

(54) **NOVEL GENOME SEQUENCING STRATEGIES**
NEUE GENOMSEQUENZIERUNGSSTRATEGIEN
NOUVELLES STRATÉGIES DE SÉQUENÇAGE DU GÉNOME

(30) Priority: 13.01.2009 US 144281 P; 17.07.2009 US 226468 P; 17.07.2009 NL 2003235
(43) Date of publication of application: 26.10.2011
(73) Proprietor: Keygene N.V., 6700 AE Wageningen (NL)
(72) Inventor: VAN EIJK, Michael, Josephus, Theresia, 5373 EJ Herpen (NL); VAN TUNEN, Adrianus, Johannes, 6708 NA Wageningen (NL); JANSSEN, Antoine, Antonius, Arnoldus, Wilhelmus, 6663 HR Lent (NL)
(74) Representative: de Lang, Robbert-Jan
(86) International application number: PCT/NL2010/000003
(87) International publication number: WO 2010/082815

(56) References cited:
- WO-A-2008/007951
- SEA URCHIN GENOME SEQUENCING CONSORTIUM ET AL: "The genome of the sea urchin Strongylocentrotus purpuratus." SCIENCE (NEW YORK, N.Y.) 10 NOV 2006, vol. 314, no. 5801, 10 November 2006 (2006-11-10), pages 941-952, XP008120409 ISSN: 1095-9203
- MILOSAVLJEVIC A ET AL: "Shotgun sequencing, clone pooling, and comparative strategies for mapping and sequencing" TARGETS, ELSEVIER, vol. 2, no. 6, 1 December 2003 (2003-12-01), pages 245-252, XP004886692 ISSN: 1477-3627

## Description

### Technical field of the invention

The present invention relates to an efficient method for *de novo* whole genome sequencing. The invention relates to large-scale nucleic acid sequencing and in particular to methods for sequencing the genome, or a part thereof, of an organism. The invention relates to improved strategies for determining the sequence of, preferably complex (i.e. large) genomes, based on the use of high throughput sequencing technologies.

### Background of the invention

The goal of many sequencing projects is to determine, for the first time, the entire genome sequence of a target organism (*de novo* draft genome sequencing). Having a draft genome sequence at hand enables identification of useful genetic information of an organism, for instance for the identification of the origin of genetic variety between species or individuals of the same species. Hence, it is a general desire in the art to come to techniques that allow the *de novo* determination of the entire genome sequence of an individual whether human, animal or plant at a reasonable cost and effort. This quest is typically indicated as the quest for the 1000$-genome, i.e. determining the entire genome sequence of an individual for a maximum of 1000$ (without considering currency fluctuations). However, in practice the 1000$ genome does not necessarily rely on *de novo* genome sequencing and assembly strategy but may also be based on a re-sequencing approach. In case of the latter, the re-sequenced genome will not be assembled *de novo,* but its DNA sequenced compared to (mapped onto) an existing reference genome sequence for the organism of interest. A re-sequencing approach is therefore technically less challenging and less costly. For sake of clarity, the focus of the current invention is on *de novo* genome sequencing strategies, capable to be applied to organisms for which a reference genome sequence is lacking.

Current efforts are varying, plentiful and rapidly increasing results are achieved. Nevertheless, the goal has not been achieved yet. It is still not economically feasible to sequence and assemble an entire genome in a straight forward fashion. There exists still a need in the art for improved *de novo* genome sequencing strategies.

WO03/027311 describes a clone-array pooled shotgun sequencing method (CAPPS). The method employs random sequence reads from differently pooled (BAC) clones. Based on the cross-assembly of the random reads a sequence contig can be generated from a plurality of clones and a map of the clones relative to the sequence can be generated. The publication describes, in more detail, the generation of a BAC library in multidimensional pools, for example a two-dimensional format where each pool and row contain 148 BAC clones (148 x 148 format). Using CAPPS, BAC pools are sequenced to 4-5 X coverage on average, which generates 8-10X coverage per BAC In case of the two-dimensional pooling scheme. The contigs are made per BAC separately based on sequences that are unique to the BAC based on their occurrence in a single row and an single pool in case of a two-dimensional pooling scheme. Subsequently these BACs are assembled in a contig for the genome. The publication demonstrates the technology based on 5 BACs only. The publication leaves the problem of data-processing untouched. However, one of the disadvantages of this technology is that the use of randomly sheared fragments requires an enormous amount of reads to cover a genome at a sequence redundancy level of 8 to 10 fold, making this method very laborious on larger scale. Furthermore it does not yield a sequence based physical BAC map.

US2007/0082358 describes a method of *de novo* assembly of sequence information based on a clonally isolated and amplified library of single stranded genomic DNA to create whole genome shotgun sequence information combined with whole genome optical restriction mapping using a restriction enzyme for the creation of an ordered restriction map.

US2002/0182830 discloses a method on BAC contig mapping by comparison of subsequences. The method aims at avoiding the difficulties associated with repetitive sequences and the generation of contigs by the creation of bridges across repeat-rich regions.

Determining physical maps based on BACs can be based on sequencing BAC libraries (sequence-based physical mapping of BAC clones) using for instance the method described in WO2008/00795 from Keygene also indicated as 'whole genome profiling' or WGP. In brief, WGP relates to the generation of a physical map of at least part of a genome comprising the steps of generating an artificial chromosome library from a sample DNA, pooling the clones, digesting the pooled clones with restriction enzymes, ligating identifier-containing adapters, amplifying the identifier-containing adapter-ligated restriction fragments, correlating the amplicons to the clones and ordering the fragments to generate a contig to thereby create a physical map.

WO2008/007951 describes a method for the determination of physical maps based on BAC clones using the WGS methodology.

The Sea Urchin Genome Sequencing Consortium (Science (2006) Vol. 314:941-952) describes a method to sequence the whole Genome of Sea Urchin using two different strategies, CAPSS and WGS, which are combined to assemble the Sea Urchin Genome.

Despite all developments in high throughput sequencing, determining draft genome sequences with high accuracy is still considered expensive and laborious. There remains a need to complement the currently existing methods to come to efficient and economic methods for the generation of draft genome sequences. In particular, the current high throughput sequencing technologies provide relative short reads (up to 400 nt), resulting in relative short contigs which are difficult to assemble in to larger contigs and puts a high demand on computational power.

### Summary of the invention

The present inventors have found that combining clone-based whole genome profiling with (high throughput) sequencing of fragments of sample (genomic) DNA using high throughput sequencing technologies provides for a superior strategy for the determination of draft genome sequences with high accuracy and speed. By generating contigs from the sequencing reads and anchoring these reads to the BAC (or YAC or any other large insert cloning vector)-contig obtained via whole genome profiling, contigs are generated of increased length and density. Hence a draft genome sequence is obtained which is generated by a reduced number of contigs thereby increasing the quality thereof.

### Definitions

Clustering: with the term "clustering" is meant the comparison of two or more nucleotide sequences based on the presence of short or long stretches of identical or similar nucleotides and grouping together the sequences with a certain minimal level of sequence homology based on the presence of short (or longer) stretches of identical or similar sequences.

Alignment: positioning of multiple sequences in a tabular presentation to maximize the possibility for obtaining regions of sequence identity across the various sequences in the alignment, e.g. by introducing gaps Several methods for alignment of nucleotide sequences are known in the art, as will be further explained below.

AFLP: AFLP refers to a method for selective amplification of nucleic acids based on digesting a nucleic acid with one or more restriction endonucleases to yield restriction fragments, ligating adaptors to the restriction fragments and amplifying the adaptor-ligated restriction fragments with at least one primer that is (part) complementary to the adaptor, (part) complementary to the remains of the restriction endonuclease, and that may further contain at least one randomly selected nucleotide from amongst A,C, T, or G (or U as the case may be) at the 3'end of the primer. AFLP does not require any prior sequence information and can be performed on any starting DNA. In general, AFLP comprises the steps of:
(a) digesting a nucleic acid, in particular a DNA or cDNA, with one or more specific restriction endonucleases, to fragment the DNA into a corresponding series of restriction fragments;
(b) ligating the restriction fragments thus obtained with a double-stranded synthetic oligonucleotide adaptor, one end of which is compatible with one or both of the ends of the restriction fragments, to thereby produce adaptor-ligated, restriction fragments of the starting DNA;
(c) contacting the adaptor-ligated, restriction fragments under hybridizing conditions with one or more oligonucleotide primers that are directed towards the adapters and that may contain selective nucleotides at their 3'-end;
(d) amplifying the adaptor-ligated, restriction fragment hybridised with the primers by PCR or a similar technique so as to cause further elongation of the hybridised primers along the restriction fragments of the starting DNA to which the primers hybridised; and
(e) detecting, identifying or recovering the amplified or elongated DNA fragment thus obtained.

AFLP thus provides a reproducible subset of adaptor-ligated fragments. AFLP is described in EP 534858, US 6045994 and in Vos et al 1995. AFLP: a new technique for DNA fingerprinting. Nucleic Acids Research 23(21):4407-4414. Reference is made to these publication for further details regarding AFLP. The AFLP is commonly used as an efficient, robust and reproducible complexity reduction technique.

Selective base or selective nucleotide: Located at the 3' end of the primer that contains a part that is complementary to the adaptor and a part that is complementary to the remains of the restriction site, the selective base is randomly selected from amongst A, C, T or G (or U as the case may be). By extending a primer with a selective base, the subsequent amplification will yield only a reproducible subset of the adaptor- ligated restriction fragments, i.e. only the fragments that can be amplified using the primer carrying the selective base. Selective nucleotides can be added to the 3'end of the primer in a number varying between 1 and 10. Typically, 1-4 suffice. Both primers may contain a varying number of selective bases. With each added selective base, the subset reduces the amount of amplified adaptor-ligated restriction fragments in the subset by a factor of about 4. Typically, the number of selective bases used in AFLP is indicated by +N+M, wherein one primer carries N selective nucleotides and the other primers carries M selective nucleotides. Thus, an EcoRI/MseI +1/+2 AFLP is shorthand for the digestion of the starting DNA with EcoRI and Msel, ligation of appropriate adaptors and amplification with one primer directed to the EcoRI restricted position carrying one selective base and the other primer directed to the Msel restricted site carrying 2 selective nucleotides. A primer used in AFLP that carries at least one selective nucleotide at its 3' end is also depicted as an AFLP-primer. Primers that do not carry a selective nucleotide at their 3' end and which in fact are complementary to the adaptor and the remains of the restriction site are sometimes indicated as AFLP+0 primers. The term selective nucleotide is also used for nucleotides of the target sequence that are located adjacent to the adaptor section and that have been identified by the use of selective primer as a consequence of which, the nucleotide has become known.

Sequencing: The term sequencing refers to determining the order of nucleotides (base sequences) in a nucleic acid sample, e.g. DNA or RNA. Many techniques are available such as Sanger sequencing and high-throughput sequencing technologies (also known as next-generation sequencing technologies) such as the GS FLX platform offered by Roche Applied Science, based on pyrosequencing.

Restriction endonuclease: a restriction endonuclease or restriction enzyme is an enzyme that recognizes a specific nucleotide sequence (target site) in a double-stranded DNA molecule, and will cleave both strands of the DNA molecule at or near every target site, leaving a blunt or a staggered end.

Frequent cutters and rare cutters: Restriction enzymes typically have recognition sequences that vary in number of nucleotides from 3, 4 (such as Msel) to 6 (EcoRI) and even 8 (Notl). The restriction enzymes used can be frequent and rare cutters. The term 'frequent' in this respect is typically used in relation to the term 'rare'. Frequent cutting endonucleases (aka frequent cutters) are restriction endonucleases that have a relatively short recognition sequence. Frequent cutters typically have 3-5 nucleotides that they recognise and subsequently cut. Thus, a frequent cutter on average cuts a DNA sequence every 64-1024 nucleotides. Rare cutters are restriction endonucleases that have a relatively long recognition sequence. Rare cutters typically have 6 or more nucleotides that they recognise and subsequently cut. Thus, a rare 6-cutter on average cuts a DNA sequence every 4096 nucleotides, leading to longer fragments. It is observed again that the definition of frequent and rare is relative to each other, meaning that when a 4 bp restriction enzyme, such as Msel, is used in combination with a 5-cutter such as Avall, Avall is seen as the rare cutter and Msel as the frequent cutter.

Restriction fragments: the DNA molecules produced by digestion with a restriction endonuclease are referred to as restriction fragments. Any given genome (or nucleic acid, regardless of its origin) will be digested by a particular restriction endonuclease into a discrete set of restriction fragments. The DNA fragments that result from restriction endonuclease cleavage can be further used in a variety of techniques and can for instance be detected by gel electrophoresis.

Ligation: the enzymatic reaction catalyzed by a ligase enzyme in which two double-stranded DNA molecules are covalently joined together is referred to as ligation. In general, both DNA strands are covalently joined together, but it is also possible to prevent the ligation of one of the two strands through chemical or enzymatic modification of one of the ends of the strands. In that case the covalent joining will occur in only one of the two DNA strands.

Synthetic oligonucleotide: single-stranded DNA molecules having preferably from about 10 to about 50 bases, which can be synthesized chemically are referred to as synthetic oligonucleotides. In general, these synthetic DNA molecules are designed to have a unique or desired nucleotide sequence, although it is possible to synthesize families of molecules having related sequences and which have different nucleotide compositions at specific positions within the nucleotide sequence. The term synthetic oligonucleotide will be used to refer to DNA molecules having a designed or desired nucleotide sequence.

Adaptors: short double-stranded DNA molecules with a limited number of base pairs, e.g. about 10 to about 30 base pairs in length, which are designed such that they can be ligated to the ends of restriction fragments. Adaptors are generally composed of two synthetic oligonucleotides which have nucleotide sequences which are partially complementary to each other. When mixing the two synthetic oligonucleotides in solution under appropriate conditions, they will anneal to each other forming a double-stranded structure. After annealing, one end of the adaptor molecule is designed such that it is compatible with the end of a restriction fragment and can be ligated thereto; the other end of the adaptor can be designed so that it cannot be ligated, but this need not be the case (double ligated adaptors).

Adaptor-ligated restriction fragments: restriction fragments that have been capped by adaptors.

Primers: in general, the term primers refer to DNA strands which can prime the synthesis of DNA. DNA polymerase cannot synthesize DNA de novo without primers: it can only extend an existing DNA strand in a reaction in which the complementary strand is used as a template to direct the order of nucleotides to be assembled. We will refer to the synthetic oligonucleotide molecules which are used in a polymerase chain reaction (PCR) as primers.

DNA amplification: the term DNA amplification will be typically used to denote the in vitro synthesis of double-stranded DNA molecules using PCR. It is noted that other amplification methods exist and they may be used in the present invention without departing from the gist.

Nucleic acid: a nucleic acid according to the present invention may include any polymer or oligomer of pyrimidine and purine bases, preferably cytosine, thymine, and uracil, and adenine and guanine, respectively (*See* Albert L. Lehninger, Principles of Biochemistry, at 793-800 (Worth Pub. 1982)). The present invention contemplates any deoxyribonucleotide, ribonucleotide or peptide nucleic acid component, and any chemical variants thereof, such as methylated, hydroxymethylated or glycosylated forms of these bases, and the like. The polymers or oligomers may be heterogenous or homogenous in composition, and may be isolated from naturally occurring sources or may be artilicially or synthetically produced. In addition, the nucleic acids may be DNA or RNA, or a mixture thereof, and may exist permanently or transitionally in single-stranded or double-stranded form, including homoduplex, heteroduplex, and hybrid states.

Complexity reduction: the term complexity reduction is used to denote a method wherein the complexity of a nucleic acid sample, such as genomic DNA, is reduced by the generation or selection of a subset of the sample. This subset can be representative for the whole (i.e. complex) sample and is preferably a reproducible subset. Reproducible means in this context that when the same sample is reduced in complexity using the same method and experimental conditions, the same, or at least comparable, subset is obtained. The method used for complexity reduction may be any method for complexity reduction known in the art. Examples of methods for complexity reduction include for example AFLP® (Keygene N.V., the Netherlands; see e.g. EP 0 534 858), the methods described by Dong (see e.g. WO 03/012118, WO 00/24939), indexed linking (Unrau et al., vide infra), etc. The complexity reduction methods used in the present invention have in common that they are reproducible. Reproducible in the sense that when the same sample is reduced in complexity in the same manner, the same subset of the sample is obtained, as opposed to more random complexity reduction such as microdissection, random shearing, or the use of mRNA (cDNA) which represents a portion of the genome transcribed in a selected tissue and for its reproducibility is depending on the selection of tissue, time of isolation etc..

Tagging: the term tagging refers to the addition of a sequence tag to a nucleic acid sample in order to be able to distinguish it from a second or further nucleic acid sample. Tagging can e.g. be performed by the addition of a sequence identifier during complexity reduction or by any other means known in the art such as a separate ligation step. Such a sequence identifier can e.g. be a unique base sequence of varying but defined length uniquely used for identifying a specific nucleic acid sample. Typical examples are ZIP sequences, known in the art as commonly used tags for unique detection by hybridization (lannone et al. Cytometry 39:131-140, 2000). Using nucleotide based tags, the origin of a sample, a clone or an amplified product can be determined upon further processing. In case of combining processed products originating from different nucleic acid samples, the different nucleic acid samples should be identified using different tags.

Identifier: a short sequence that can be added to an adaptor or a primer or included in its sequence or otherwise used as label to provide a unique identifier (aka barcode or index). Such a sequence identifier (tag) can be a unique base sequence of varying but defined length, typically from 4-16 bp used for identifying a specific nucleic acid sample. For instance 4 bp tags allow 4(exp4) = 256 different tags. Using such an identifier, the origin of a PCR sample can be determined upon further processing or fragments can be related to a clone. Also clones in a pool can be distinguished from one another using these sequence based identifiers. Thus, identifiers can be sample specific, pool specific, clone specific, amplicon specific etc. In the case of combining processed products originating from different nucleic acid samples, the different nucleic acid samples are generally identified using different identifiers. Identifiers preferably differ from each other by at least two base pairs and preferably do not contain two identical consecutive bases to prevent misreads. The identifier function can sometimes be combined with other functionalities such as adapters or primers and can be located at any convenient position.

Tagged library: the term tagged library refers to a library of tagged nucleic acids.

Aligning and alignment: With the term "aligning" and "alignment" is meant the comparison of two or more nucleotide sequence based on the presence of short or long stretches of identical or similar nucleotides. Several methods for alignment of nucleotide sequences are known in the art, as will be further explained below.

The term "contig" is used in connection with DNA sequence analysis, and refers to assembled contiguous stretches of DNA derived from two or more DNA fragments having contiguous nucleotide sequences. Thus, a contig is a set of overlapping DNA fragments that provides a partial contiguous sequence of a genome. A "scaffold" is defined as a series of contigs that are in the correct order, but are not connected in one continuous sequence , i.e. contain gaps. Contig maps also represent the structure of contiguous regions of a genome by specifying overlap relationships among a set of clones. For example, the term "contigs" encompasses a series of cloning vectors which are ordered in such a way as to have each sequence overlap that of its neighbours. The linked clones can then be grouped into contigs, either manually or, preferably, using appropriate computer programs such as FPC, PHRAP, CAP3 etc.

The term 'scaffold' is used for contigs generated *inter alia* via paired end sequencing that contains gaps of (un)known size. The term 'superscaffold' is used for scaffolds thar are linked to each other via WGP BAC contigs.

High-throughput screening: High-throughput screening, often abbreviated as HTS, is a method for scientific experimentation especially relevant to the fields of biology and chemistry. Through a combination of modern robotics and other specialised laboratory hardware, it allows a researcher to effectively screen large amounts of samples simultaneously.

Upstream or downstream: A convention used to describe features of a DNA sequence in terms of the direction (5' to 3') of the DNA sequence. Downstream (or 3' to) is in the direction of the 3'end of the DNA sequence, whereas upstream (5' to) is in the direction of the 5'-end of the DNA sequence. Conventionally single stranded DNA sequences, gene maps and RNA sequences are drawn with transcription (or translation) from left to right and so downstream is towards the right (and upstream to the left). The term downstream or upstream can be used to define relative positions of various DNA segments to each other in a DNA sequence. For instance in an AFLP fragment, the selective nucleotide in the fragment is located upstream from the adapter, but the selective nucleotide in the primer is located downstream (i.e. 3' to) the adapter-complementary section of the primer.

### Description of the Drawings

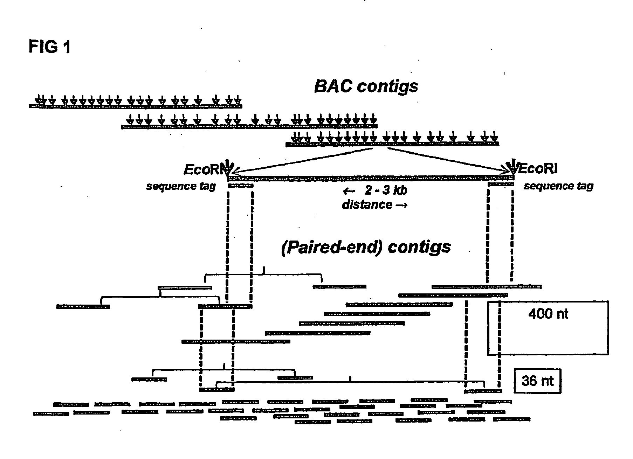

Figure 1 is a visual representation of combining whole genome profiling and whole genome sequencing using BAC derived sequences and shotgun sequencing to generate contigs and scaffolds.

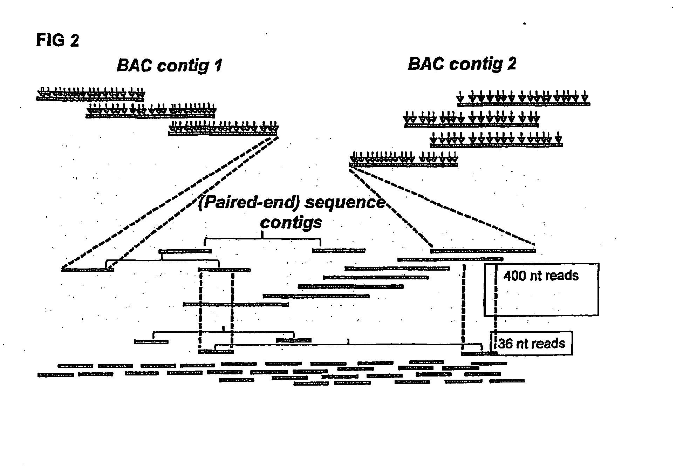

Figure 2 is a visual representation of combining whole genome profiling and whole genome sequencing using BAC derived sequences and shotgun sequencing to complement BAC-derived contigs and to fill gaps between BAC contigs.
Figure 3 is a visualization of a contig size distribution obtained in the generation of BAC derived contigs for melon.
Figure 4 is a visual representation of the primer structure and its interaction with the adapter and the identifier
Figure 5. is a visual representation of scaffold generation. Blocks are the BAC contigs, horizontal lines are WGS scaffolds and vertical lines are linked tags
Figure 6. is a visual representation of branched scaffold generation. Blocks are the BAC contigs, horizontal lines are WGS scaffolds and vertical lines are linked tags. The horizontal line in dotted detail shows another WGS scaffold linked to the same BAC contig, which results in two branches.

### Detailed description of the invention

The present inventors have found a novel (plant) genome sequencing strategy and applied this to a commercial vegetable crop (melon). This genome sequencing strategy is based on two components:
1) construction of a sequence-based physical map, preferably by sequencing fragment ends of pooled artificial chromosomes (preferably BAC) clones (Amplicon Express, Pullman, USA), preferably using the Genome Analyzer II and
2) Whole genome sequencing (WGS), preferably comprising a combination of single reads, 3 kb paired-end reads and long-jump paired-end reads using the GS FLX Titanium or GA II.

Maximum assembly power is obtained when both the sequence-based physical map and the WGS sequences are generated using the same (homozygous/inbred) line, as was the case for the crop described in the appended examples.

Thus, in a first aspect, the invention pertains to a method for the determination of a genome sequence comprising the steps of:
- providing a physical map of a sample genome by sequencing fragment ends of pooled BAC clones;
- providing a set of sequence reads from the sample DNA;
- generating a contig of the physical map and the sequence reads.

In this way an efficient and high quality draft of the genome sequence can be obtained as the sequence reads complement the scaffold provided for by the physical map obtained via the contig of the sequenced fragment ends of the clones.

The invention in one embodiment pertains to a method for the determination of a genome sequence comprising the steps of:
(a) providing a sample DNA;
(b) generating an artificial chromosome (e.g. BAC, YAC) clone bank wherein each artificial chromosome clone contains part of the sample DNA;
(c) combining the artificial chromosome clones in one or more pools, wherein each clone is present in more than one pool, to create a library;
(d) providing a set of fragments for each pool;
(e) ligating adaptors to one or both sides of the fragments,
(f) determining the sequence of at least part of the adaptor and part of the fragment;
(g) assigning the fragment sequences to the corresponding clones ;
(h) building a clone-contig thereby generating a physical map of the sample genome;
(i) generating sequence reads from a sample DNA;
(j) aligning the sequence reads and/or contigs or scaffolds from the sequence reads to the clone contig to thereby build a genome sequence/super scaffold.

This strategy combines the power of BAC-based physical mapping with whole genome sequencing. The method according to the invention provides significant cost reductions compared to the currently used genome sequencing strategies. The method further provides for increased flexibility to combine sequence information derived from artificial chromosomes such as BAC-derived sequences and sequence information derived from techniques that directly generate sequence information such as whole genome shotgun sequencing and similar techniques. The present method can also be supplemented with other sequence information available such as obtained via more conventional technique as Sanger didexoysequencing.

In step (a) of the method a sample DNA is provided. This can be achieved by any means in the art such as disclosed for instance by Sambrook et al (Sambrook and Russell (2001) "Molecular Cloning: A Laboratory Manual (3rd edition), Cold Spring Harbor Laboratory, Cold Spring Harbor Laboratory Press). The sample DNA can be from any species, in particular from human, plant or animal origin. It is possible to use only a part of a genome, but that is not necessary as the present invention also provides for methods to accommodate genomes of any size, for instance through the creation of reproducible subsets via reproducible complexity reduction such as for instance selective amplification based on AFLP (EP534858). Thus typically, the present method uses the entire genome.

In step (b) an artificial clone bank is generated. The library can be a Bacterial Artificial Chromosome library (BAC) or based on yeast (YAC). Other libraries such as based on fosmids, cosmids, PAC, TAC or MAC are also possible. Preferred is a BAC library. The library is preferably of a high quality and preferably is a high insert size genomic library. This means that the individual BAC contains a relative large insert of the genomic DNA under investigation (typically > 125 kbp). The size of the preferred large insert is species-dependent. Throughout this application reference can be made to BACs as examples of artificial chromosomes. However, it is noted that the present invention is not limited thereto and that other artificial chromosomes can be used without departing from the gist of the invention. Preferably the libraries contain at least five genome equivalents, more preferably at least 7, most preferably at least 8. Particularly preferred is at least 10. The higher the number of genome equivalents in the library, the more reliable the resulting contigs and physical map will be.

The individual clones in the library are pooled to form pools containing a multitude of artificial chromosomes or clones. The pooling may be the simple combination of a number of individual clones into one sample (for example, 100 clones into 10 pools, each containing 10 clones), but also more elaborate pooling strategies may be used. The distribution of the clones over the pools is preferably such that each clone is present in at least one or two or more pools thereby generating a library. Preferably, the pools contain from 10 to 10000 clones per pool, preferably from 100 to 1000, more preferably from 250 to 750. It is observed that the number of clones per pool can vary widely, and this variation is related to, for instance, the size of the genome under investigation. Typically, the maximum size of a pool or a sub-pool is governed by the ability to uniquely identify a clone in a pool by a set of identifiers. A typical range for a genome equivalent in a pool is in the order of 0.2 - 0.3, and this may again vary per genome. The pools are generated based on pooling strategies well known in the art. The skilled man is capable selecting the optimal pooling strategy based on factors such as genome size etc. The resulting pooling strategy will depend on the circumstances, and examples thereof are plate pooling, N-dimensional pooling such as 2D-pooling, 3D-pooling, 6D-pooling or complex pooling. To facilitate handling of large numbers of pools, the pools may, on their turn, be combined in super-pools (i.e. super-pools are pools of pools of clones) or divided into sub-pools. Other examples of pooling strategies and their deconvolution (i.e. the correct identification of the individual clone in a library by detection of the presence of a known associated indicator (i.e. label or identifier) of the clone in one or more pools or subpools) are for instance described in US 6975943 or in Klein et al. in Genome Research, (2000), 10, 798-807. The pooling strategy is preferably such that every clone in the library is distributed in such over the pools that a unique combination of pools is made for every clone. The result thereof is that a certain combination of (sub)pools uniquely identifies a clone.

In step (d) of the method the pools are fragmented and a set of fragments is produced or each pool. The fragmentation can be randomly, i.e. via shearing or nebulisation to create a set of fragments. In a preferred embodiment, the pools are digested with restriction endonucleases to yield restriction fragments. Each pool is, preferably separately, subjected to an endonuclease digest. Each pool is treated with the same (combination of) endonuclease(s). In principle, any restriction endonuclease can be used. Restriction endonucleases may be frequent cutters (4 or 5 cutters, such as Msel or Avall) or rare cutters (6 and more cutters such as EcoRI, HindIII, PacI). Typically, restriction endonucleases are selected such that restriction fragments are obtained that are, on average, present in an amount or have a certain length distribution that is adequate for the required profiling resolution and/or subsequent steps. In certain embodiments, two or more restriction endonucleases can be used and in certain embodiments, combinations of rare and frequent cutters can be used. For large genomes the use of, for instance, three or more restriction endonucleases can be used advantageously. In certain embodiments, restriction enzymes can be used that provide for blunt ends. The corresponding adaptors (see below) then may also be blunt ended.

To one or both ends of the fragments, adaptors are ligated in step (e) to provide for adaptor-ligated fragments. Typically, adaptors are synthetic oligonucleotides as defined herein elsewhere. The adaptors used in the present invention preferably contain an identifier section, in essence as defined herein elsewhere. In certain embodiments, the adaptor contains a pool-specific identifier, i.e. for each pool, an adapter containing a unique identifier is used that unequivocally indicates the pool from which the fragment originates. In certain embodiments, the adaptor contains a degenerate identifier section, which is used in combination with a primer containing a pool-specific identifier. The adapter may further contain primer binding sites on which later on amplification may be initiated. These primer-binding sites may also be ligated at a later stage. It is preferred that the identifier section (degenerate or not) is located between the fragment and the primer binding site such that amplification from the primer binding site using primers complementary to the primer binding site at least amplifies the identifier.

In certain embodiments, the adapter-ligated fragments can be combined in larger groups, in particular when the adaptors contain a pool-specific identifier. This combination in larger groups may aid in reducing the number of parallel amplifications of each set of adapter-ligated restriction obtained from a pool.

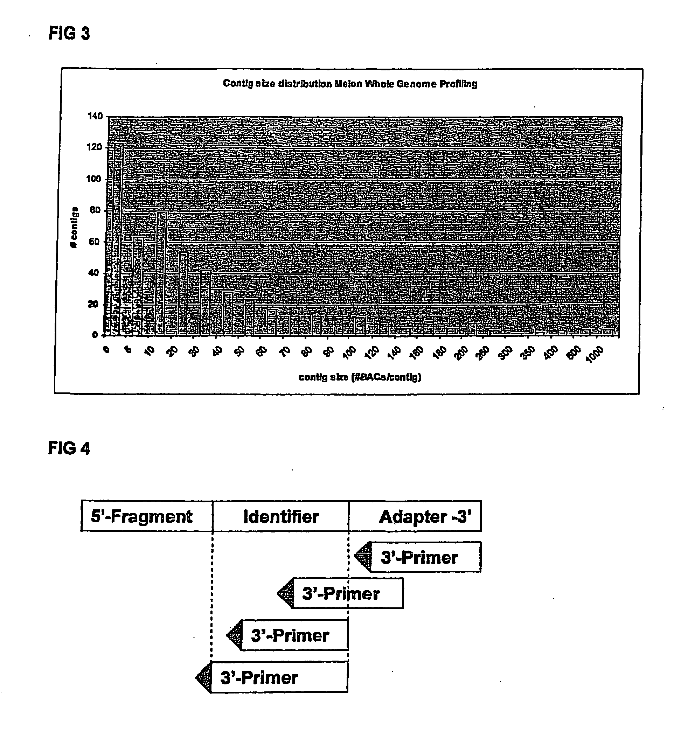

The adaptor-ligated fragments can be amplified using a set of primers of which at least one primer amplifies the pool-specific identifier at the position of the pool-specific or degenerate identifier in the adaptor. The primer may contain (part of) the identifier, but the primer may also be complementary to a section of the adapter that is located outside the identifier, i.e. downstream in the adapter. Amplification then also amplifies the identifier (see also fig 4). In one embodiment, the primer may contain an identifier at a position located 5' from the part that is complementary to the adapter so that amplification introduces the identifier in the resulting amplicon

This embodiment also allows for the grouping of adaptor-ligated fragments prior to the amplification as outlined above. In an alternative embodiment, each pool of adaptor-ligated fragments, wherein the adaptor contained a degenerate identifier section, is amplified separately using a set of primers of which at least one primer contains a pool-specific section that serves as an identifier, thereby uniquely identifying the pool. In another embodiment, the primer that is complementary to at least part of the adapter and provides an identifier in the amplified adapter-ligated fragment, for instance by containing at the 5'end of the part that is complementary to the adaptor, an identifier sequence. Amplification with this primer adds an identifier to the amplicon.

Either way, the result is a set of amplified adapter-ligated fragments, also depicted as amplicons, that are linked to the pool from which they originate by the presence in the amplicon of the pool-specific identifier. In certain embodiments, sub-sets of amplicons may be created by selective amplification for instance by using primers carrying selective nucleotides at their 3' end, essentially as described herein elsewhere.

The amplicons may be combined in certain embodiments, in a set of combined amplicons or a so-called sequence library.

In step (f) of the method, the fragments are (or, when amplified, the amplicons) are subjected to sequencing, preferably high throughput sequencing as described herein below. During sequencing, at least part of the nucleotide sequence of the adaptor-ligated fragment is determined. Preferably, at least the sequence of part of the adaptor and part of the fragment sequence is determined. Preferably, the sequenced part allows for correlation of the sequence to the BAC clone. Preferably, the sequence of the pool-specific identifier and part of the fragment (i.e. derived from the sample genome) is determined. Preferably, a sequence of at least 10 nucleotides of the fragment is determined. In certain embodiments, at least 11, 12, 13, 14 or 15 nucleotides of the fragment are determined. The number of nucleotides that are to be determined minimally will be, again, genome- as well as sequencing platform dependent. For instance, in plants more repetitive sequences are present, hence longer sequences (25-75 nucleotides) are to be determined for a contig of comparable quality. For instance, in silico calculations on the known genome sequence of Arabidopsis have shown that, when including a 6 bp restriction site in the sequencing step, about 20 bp per fragment needs to be determined in order to ensure that the majority of sequences are unique in the genome. It is possible to determine the sequence of the entire fragment, but this is not an absolute necessity for contig building of a BAC clone.

In the sequencing step, to provide for maximum coverage of all fragments and increased accuracy, the sequence library may be sequenced with an average redundancy level (aka oversampling rate) of at least 5. This means that, on average, the sequence is determined of at least 5 amplicons obtained from the amplification of one specific adaptor-ligated fragment. In other words: each fragment is (statistically) sequenced at least five times. Increased redundancy is preferred as its improves fraction of fragments that are sampled in each pool and the accuracy of these sequences, so preferably redundancy level is at least 7, more preferably a least 10. Increased average sequencing redundancy levels are used to compensate for a phenomenon that is known as 'sampling variation', i.e. random statistical fluctuation in sampling subsets from a large "population". In addition, a higher average sequencing redundancy level alleviates possible differences in the abundance of amplified fragments which result from differences in their amplification rates caused by length variation between fragments and differences in sequence composition.

In the following step (g), the (partly) sequenced adaptor-ligated fragments or amplicons are correlated or assigned to the corresponding clone, typically in silico by means of computerized methods. The adaptor-ligated fragments or amplicons are selected that contain identical sections of nucleotides in the fragment-derived part. Subsequently the different pool-specific identifiers are identified that are present in those adaptor-ligated fragments or amplicons. The combination of the different pool-specific identifiers and hence the sequence of the fragment can be uniquely assigned to a specific clone (a process known as 'deconvolution'). For example, in the case of a 3D pooling strategy (X,Y,Z), each pool in the library is uniquely addressed by a combination of 3 pool-specific identifiers. Each clone occurs more than once in the library, so for each occurrence of a clone in the library, a combination of 3 pool-specific identifiers can be made in combination with the same fragment-derived section. In other words: a fragment-derived section originating from a clone will be tagged with 3 different identifiers. Unique fragment-derived sections, when observed in combination with the 3 identifiers can be assigned to a single BAC clone. This can be repeated for each adaptor-ligated fragments or amplicon that contains other unique sections of nucleotides in the fragment-derived part. This process of deconvolution can be made easier by keeping the genome equivalent per pool relatively low (<0.3, pref. 0.2), thereby reducing the chance that the same fragment is present twice in the same pool derived from different clones.

A sample DNA is converted into BAC library. The BAC library can be pooled in a set of pools (M) (e.g. 3 pools, each containing about 0.3 GE,). Each pool is divided into (X+Y+Z) subpools (typically a stack of microtiterplates or row and/or column pools).

The sequenced adaptor-ligated fragments or amplicons that are now linked to a particular clone in the library can be used in building a contig based on sequence matching of the fragment derived sections. The contigs of each clone can then be aligned to generate a physical map. In one embodiment, the fragments derived from the same clone can be ordered to build a contig from the clone. Based on the occurrence of the fragments sequence in two or more clones (WGP tags), the clones can be linked to each other in step (h) of the invention, thereby forming a clone contig and hence a physical map of the sample genome.

The high throughput sequencing used in the present invention is a method for scientific experimentation especially relevant to the fields of biology and chemistry. Through a combination of modern robotics and other specialised laboratory hardware, it allows a researcher to effectively screen large amounts of samples simultaneously.

It is preferred that the sequencing is performed using high-throughput sequencing methods, such as the methods disclosed in WO 03/004690, WO03/054142, WO 2004/069849, WO 2004/070005, WO 2004/070007, and WO 2005/003375, by Seo et al. (2004) Proc. Natl. Acad. Sci. USA 101:5488-93, and technologies of Helicos, Illumina), US Genomics, etcetera.

### Roche Applied Science

In certain embodiments, it is preferred that sequencing is performed using the apparatus and/or method disclosed in WO 03/004690, WO 03/054142, WO 2004/069849, WO 2004/070005, WO 2004/070007, and WO 2005/003375. At present, the technology described allows sequencing of 400,000 sequence reads in a single GS FLX Titanium run,and is 100 times faster and cheaper than competing technology. The sequencing technology essentially contains 5 steps: 1) fragmentation of DNA and ligation of specific adaptors to create a library of single-stranded DNA (ssDNA); 2) annealing of ssDNA to beads, emulsification of the beads in water-in-oil microreactors and performing emulsion PCR to amplify the individual ssDNA molecules on beads; 3) selection of /enrichment for beads containing amplified ssDNA molecules on their surface 4) deposition of DNA carrying beads in a PicoTiter™Plate; and 5) simultaneous sequencing in more than 1 million wells of a PicoTiter™Plate by generation of a pyrophosphate light signal. The method will be explained in more detail below.

In a preferred embodiment, the sequencing comprises the steps of:
a. annealing adapted fragments to beads, each bead being annealed with a single adapted fragment;
b. emulsifying and amplifying the annealed fragments on the beads in water-in-oil microreactors, each water-in-oil microreactor comprising a single bead;
c. loading the beads in wells, each well comprising a single bead; and generating a pyrophosphate signal.

In the first step (a), sequencing adaptors are ligated to fragments within the combination library. Said sequencing adaptor includes at least a region for annealing to a complementary oligonucleotide bound to a bead, a sequencing primer region and a PCR primer region. Thus, adapted fragments are obtained.

In the first step, adapted fragments are annealed to the beads, each bead annealing with a single adapted fragment. To the pool of adapted fragments, beads are added in excess as to ensure annealing of one single adapted fragment per bead for the majority of the beads (Poisson distribution). In the present invention, the adapters that are ligated to the restriction fragments obtained from the clones may comprise a section that is capable of annealing to a bead.

In a next step, the beads are emulsified in water-in-oil microreactors, each water-in-oil microreactor comprising a single bead. PCR reagents are present in the water-in-oil microreactors allowing a PCR reaction to take place within the microreactors. Subsequently, the microreactors are broken, and the beads comprising DNA (DNA positive beads) are enriched, i.e. separated from beads not containing amplified fragments.

In a following step, the enriched beads are loaded in wells, each well comprising a single bead. The wells are preferably part of a PicoTiter™Plate allowing for simultaneous sequencing of a large number of fragments.

After addition of enzyme-carrying beads, the sequence of the fragments is determined using pyrosequencing. In successive steps, the PicoTiter™Plate and the beads as well as the enzyme beads therein are subjected to different deoxyribonucleotides in the presence of conventional sequencing reagents, and upon incorporation of a deoxyribonucleotide a light signal is generated which is recorded. Incorporation of the correct nucleotide will generate a pyrosequencing signal which can be detected.

Pyrosequencing itself is known in the art and described inter alia on www.biotagebio.com; www.pyrosequencing.com / section technology. The technology is further applied in e.g. WO 03/004690, WO 03/054142, WO 2004/069849, WO 2004/070005, WO 2004/070007, and WO 2005/003375 (all in the name of 454 Life Sciences now Roche Diagnostics), and Margulies et al., nature 2005, 437, 376-380.

In the present invention, the beads are preferably equipped with primer sequences or parts thereof that are capable of being extended by polymerisation to yield bead-bound amplicons. In other embodiments, the primers used in the amplification are equipped with sequences, for instance at their 5'-end, that allow binding of the amplicons to the beads in order to allow subsequent emulsion polymerisation followed by sequencing. Alternatively, the amplicons may be ligated with sequencing adaptors prior to ligation to the beads or the surface. The sequenced amplicons will reveal the identity of the identifier and hence the combination of identifiers reveals the identity of the clone.

### Illumina technologies

One of the methods for high throughput sequencing is available from Illumina Technologies(www.illumina.com) and described inter alia in WO0006770, WO0027521, WO0058507, W00123610, WO0157248, WO0157249, W002061127, W003016565, WO03048387, W02004018497, W02004018493, WO2004050915, WO2004076692, W02005021786, W02005047301, WO2005065814, W02005068656, WO2005068089, WO2005078130. In essence, the method starts with adaptor-ligated fragments of DNA, in this particular case of adapter-ligated restriction fragments of the artificial chromosome pools as described herein elsewhere. The adaptor-ligated DNA is randomly attached to a dense lawn of primers that are attached to a solid surface, typically in a flow cell. The other end of the adaptor ligated fragment hybridizes to a complementary primer on the surface. The primers are extended in the presence of nucleotides and polymerases in a so-called solid-phase bridge amplification to provide double stranded fragments. This solid phase bridge amplification may be a selective amplification. Denaturation and repetition of the solid-phase bridge amplification results in dense clusters of amplified fragments distributed over the surface. The sequencing is initiated by adding four differently labelled reversible terminator nucleotides, primers and polymerase to the flow cell. After the first round of primer extension, the labels are detected, the identity of the first incorporated bases is recorded and the blocked 3' terminus and the fluorophore are removed from the incorporated base. Then the identity of the second base is determined in the same way and so sequencing continues.

In the present invention, the adaptor-ligated fragments or the amplicons are bound to the surface via the primer binding sequence or the primer sequence. The sequence is determined as outlined, including the identifier sequence and (part of) the fragment. Currently available technology allows for the sequencing reads lengths of a maximum of 125 bases. For the purpose of Whole Genome Profiling, a sequence read length of 36 bases by may sufficient, but this depends on genome size and sequence composition (vide infra). By economical design of the adaptors and the surface bound primers, the sequencing step reads through the sample identifier, the remains of the recognition sequence of the restriction endonuclease, any optional selective bases, and the internal sequence of the restriction fragment. For example, in case of 36 base sequence reads, when a 6 base sample identifier is used, when the remains from the rare cutter EcoRI (GAATTC) are 6 bases, when 2 selective bases are used, then the length of the internal sequence of the restriction fragment will be 36 minus 14 = of 22 bases, which can be used to uniquely identify the restriction fragment in the sample. Note that the sequence of the restriction enzyme site and the (optional) selective bases are also present in the genome, but since these sequences are in common for all restriction fragments, they do not contribute to the ability to assign the sequence reads to unique clones in the libraries.

In step (i) of the method, sequence reads are generated from a sample DNA. This may the same sample that was used in the generation of the clone bank, but it may also be another sample from the same species. Using a different origin for the sample to generate sequence reads allows the use of already existing clone banks, albeit at the expense of the quality of the genome sequence thus obtained (generating contigs may be more difficult) or the resulting genome sequence is of a lower quality or contains more gaps. From the sequence reads scaffolds or contigs may be generated by aligning them, as in step (j) and anchoring them to the clone contig to either build a superscaffold or a genome sequence.

In one embodiment of the present invention, it is possible to also use randomly generated fragments from the BACs (or other artificial chromosomes) or the pools of BACs and determining (part of) the sequence thereof, using the herein described sequencing technologies. The quality of the assembly of the contig would then even more improve as not only the BAC restriction fragment ends are linked but a contig of (a part of) the BAC can be generated. Preferably combined with the sequence reads and the contigs obtained from the sample DNA, this further increases the quality.

Thus, in a preferred embodiment, a draft genome sequence is generated from a combination of BAC derived contigs, i.e. from BAC end sequences and/ or restriction fragment sequences from BACs and/or randomly sequenced BAC clones with (contigs generated from) sequence reads from sample DNA that may have been obtained by (restriction enzyme) fragmentation.

In parallel or subsequent to the generation of the clone -contig and/or the physical map, sequence reads can be generated from a sample using a more direct approach, also indicated as 'shotgun sequencing' or 'whole genome shotgun sequencing' (WGS). In this step, sequence data is generated from a sample DNA and/or from one or more artificial chromosome clone(s) of the sample DNA. The sample may be the sample that lay afoot to the clone bank, but may also be another sample from the same species or variety and thus inherently contain a small amount of polymorphisms compared to the clone bank sample. The sequence data is typically generated via fragmentation of the sample DNA, for instance via shearing, nebulisation or restriction enzyme digestion. The fragments may or may not be adapter-ligated. The adapter may contain tags to identify the origin of the fragments or the sample using so-called identifiers. The adapter-ligated fragments may be selectively or non-selectively amplified, for instance using AFLP-based technology using adapter complementary primers which may be extended at the 3'-end with one or more selective nucleotides, essentially as described herein elsewhere. In any way, sequence reads are generated using preferably high throughput sequencing technologies such as the pyrosequencing-based sequencing technologies described herein elsewhere.

The sequence reads are then assembled in contigs and/or anchored to the contig generated from the BAC library.

In a preferred embodiment, more than one sequencing technology is used to generate the sequence reads from the sample DNA. As outlined in the drawings and the text, the different technologies provide for reads of different lengths which preferably aid in anchoring and building an extended contig.

The use of 'direct' sequence reads not only completes the BAC contig, but is also capable of filling in the gaps left over by the BAC generated contig. This is in fact one of the main advantages of the present invention. In prior strategies, the use of additional sequence data (whether newly generated or taken from known sources) is contemplated only in view of the possibility of anchoring the sequence data to the contig to fill in sequence data for the BAC contig, not in the context of linking the different clone contigs together to generate a contig (scaffold) that covers a larger part of the genome. The present invention now also provides in certain embodiment the possibility of extending the BAC-contig and fill in the gaps left between the BACs on the one hand and the sequence read generated contig(s) on the other hand, thus leading to an improved quality of the resulting draft genome, as is exemplified in the figures.

Optionally, the data from the sequence reads may also be supplemented by sequence data obtained via Sanger-dideoxysequencing techniques as this may aid further in assembling high quality contigs. Also via the so-called 'next-next generation sequencing techniques such as those from Pacific Biosciences which can deliver sequence results up to multiple kb's in length, data can be supplemented.

In obtaining the sequence read, the sample DNA may, in preferred embodiments also be submitted to more reproducible complexity reduction technologies such as for example AFLP (EP534858) and /or AFLP based strategies for sequencing complex genomes, such as for instance disclosed in WO2006/13773 wherein two different restriction enzyme combinations are used in the AFLP technology to generate a contig of sequence data.

Thus, the current invention determines a draft genome sequence based on a two-way route. The first route is generating a contig of artificial chromosome (BAC) clones using Whole Genome Profiling (WGP). By using, preferably, a random but reproducible subset of restriction fragments of the BAC pools, contigs can be generated on a relative low amount of coverage data, leading to a contig of BAC clones that can be described as 'thin' or 'low density'. Thin in that there is a relative large space between the sequenced restriction fragments that enable the contigs of the BAC being assembled with a relative economical amount of sequencing and computational power. Consequently, the fraction of the whole genome that is sequenced in the process of WGP is relatively low (as the objective of WGP is clone contiging and not whole genome sequencing).

The second route is generating/collecting sequence data of, preferably, the same (full DNA) sample using high throughput sequence machines and methods such as known from Roche Applied Science (producing up to 1kb reads) and Illumina (GS FLX) producing 36-125 nt reads, and from other vendors (such as Helicos, Intelligent Biosystems, Danaher Motion-Dover, Pacific Biosciences etc.). The sequence data can be directly anchored on the BAC contig, but may first be used to generate contigs from the sequencing data. In a subsequent step these sequence-based contigs can be anchored to the BAC contig of the first route. Additionally, the sequence data and the contigs from the sequence reads can be used to link existing BAC contigs to each other, i.e. close gaps between and in scaffolds. The advantage of combining the technology is that sequence data is obtained from the same sample using different methods that can supplement each other, as exemplified in the appended figures. It is particularly advantageous to combine WGP with two or more different (high throughput) sequencing technologies. One of the particular advantages of the herein disclosed strategies, and as opposed to the prior art strategies that very often rely on brute force approaches such as WO 03/027311, is that relatively small datasets are used which are then combined.

The sequencing of the DNA obtained from a (full DNA) sample can be based on complexity-reduced representations of the full DNA , e.g. by using restriction endonuclease-digested DNA resulting in restriction fragments that may be tagged ('bar-coded') to indicate their origin when necessary. These restriction fragments can then be subjected to sequencing using, preferably, the high throughput sequencing technologies such as those mentioned herein elsewhere. Other forms of complexity reduction can be considered as well, including, but not limited to random fragmentation (by nebulization, sonication, shearing or other mechanical means) followed by size selection of fragments in a particular size range, Cot selection (based on differential hybridization kinetics of unique versus repeated sequences) or other methods for complexity reduction, In principle, the use of restriction fragments, for instance those obtained by restriction of the full DNA with rare cutters such as EcoRI, typically of a length of 2-3Kb (in AT-rich genomes), and determining the nucleotide sequences of the ends of the restriction fragments (typically 30-400 bp per end, depending on the sequencing technology used) may be sufficient to create a contig and to anchor these fragment to the WGP contig (the physical map). It will be clear that other restriction endonucleases can be used (such as frequent cutters, e.g. Msel) as well or that combinations of restriction endonucleases can be used (e.g. EcoRI/MseI). It is preferred to create contigs from the sequence data (relatively short fragments) obtained from the full DNA and subsequently anchor these (relatively longer) contigs to the available BAC contig instead of immediately anchoring the sequencing reads to the BAC contig. Again, the advantage resides in the use of relatively smaller subsets of data that allow for more efficiency in the 'data-crunching' and hence less heavy requirements in terms of computational power. Such an approach may advantageously also allow the computations or parts or elements thereof to be performed on a desktop or laptop computer in stead of heave duty servers and mainframes. Another advantage of this two route approach resides in the use of full DNA as the second source of sequences information (vis-a-vis the use of the BAC library as the first source). A BAC library always lacks full and complete coverage of a genome. By using full DNA as an additional source of DNA it becomes possible and advantageously to achieve or at least to near full coverage of the genome under investigation.

Examples of such restriction-fragment based sequencing is described for instance in WO2006/13773 which describes the use of AFLP as a complexity reduction technology in combination with high throughput sequencing to also create high quality draft genome sequences. So, in this embodiment, BAC contigs are generated as outlined herein above and combined with the contigs derived from executing the method described in WO2006/137734

In an alternative embodiment, the sequencing of the DNA based on the full DNA can be based on 'random sequence tags'. In combination with the high throughput systems known from, inter alia, Illumina, the generated sequence information can also be anchored on the BAC contig obtained from the WGP. The embodiment is derived from the realisation that the BAC is the ultimate 'paired end' The advantage from this technology resides in the fact that 'deep sequencing' (i.e. sequencing several genome equivalents (GEs) in order to obtain a higher quality of the data) is not essential anymore for obtaining a high quality genome assembly, as the main ordering of the genome has already been provided by the BAC contig (and the sequence data are mainly used to fill in the voids in the BAC contig). The present methodology therefore allows the use of less sequence data (via 'less deep sequencing' , i.e. sequencing one or only a few GEs) without affecting assembly quality. This results in a more economical process as 'less deep sequencing' is inherently more cost-effective than deep sequencing. In case for certain areas high quality sequence data are necessary, deep sequencing can be performed in selected regions by selecting certain BAC clones or BAC contigs.

Thus, one aspect of the invention pertains to the use of the herein described method for the selective sequencing of part of a genome or selected genomic region, preferably at variable coverage levels.

Compared to WO03/027311 the present method differs in that the subsets are made based on restriction fragments or on restriction fragments in combination with random shearing and not on random shearing alone. Furthermore, and contrary to WO03/027311 the sequencing of the restriction fragments is based on a very low coverage. Based on this low coverage a BAC contig is generated that is very 'thin', i.e. contains a relatively low amount of data. This 'thin' contig is then supplemented with the data obtained from the sequence reads. This is a more efficient method for the generation of the physical map and makes a more efficient use of (limited) computational power for projects of this scale.

The results of generating the draft genome can be provided as a separate product comprising, optionally in a digital format:
- the sequence data associated with the BAC library and the associated the BAC contigs;
- the sequence data associated with the sequencing of the full DNA and the associated contigs;
- software to display the BAC contigs, the DNA contigs, the combined contigs and the draft genome sequence, from an overall draft genome sequence level to the level of the nucleotides and the overlap between two fragments
- software to generate contigs from the separate sequence data
- an application to display molecular markers on the different contigs and maps
- software to visualise data quality and gaps in the sequence.

The product can be provided on a laptop equipped with a flash memory or a harddisk, a read-only data carrier such as a CD-ROM or DVD or the like. Alternatively, the product can be provided in the form of a web-based server whereby the product is provided in a digital format on a, preferably secure, server

Thus, an exemplary product can contain one or more of the following components:
a) An assembled physical map (Whole Genome Profiling; WGP).
   The map may be assembled using contig building software such as FingerPrinted Contigs (FPC) software adapted fro use with sequences instead of band mobilities. The contig can be build based on nucleotide sequences derived from pooled clones, such as BAC clones that have been assigned to individual clones by deconvolution based on identifier sequences;
b) Assemblies, including contigs, supercontigs and/or scaffolds of Whole Genome Sequencing (WGS).
   The assemblies can be generated using genome assembly software packages such as Newbler (454 Life Sciences/ Roche Applied Sciences and Short Oligonucleotide Analysis Package (SOAP) de novo (http://soap.genomics.org.cn), based on next-generation sequencing (i.e. high throughput pyrosequencing) and/or Sanger sequencing data;
c) A draft genome sequence.
   The draft genome sequence can be based on the integration of WGP (the map and data under (a)) and WGS (the data under (b). The draft genome sequence can be provided in various formats including fasta and tab delimited files;
d) Visualization software.
   Visualisation software such as and FPC to view WGP and WGS assemblies, sequences and associated clone as well as combinations thereof;
e) Sequence data.
   The actual sequence data that have been used in the he generation of the physical map or the whole genome sequencing. This may aid in further improvement of the data, for verification of the data, for allowing the generation of an improved physical map, for instance based on obtaining additional data.
f) A storage device or data carrier.
   The device or carrier can be a hard-drive or flash disk comprising one or more of the data and software described in (a )to (f);
g) A computer such as a Laptop or Netbook comprising one or more of components (a) to (f) or part thereof.

### Examples

### Arabidopsis thaliana ecotype Columbia

A BAC library was used containing 6144 BACs (about 5 genome equivalents)

One Illumina Classic run was performed on restriction enzyme (EcoRI and Msel)-fragmented pools, resulting in approximately 65,000 distinct deconvolutable sequence reads from the EcoRI side. Assembly of the reads (FPC, Soderlund, C., S. Humphrey, A. Dunhum, and L. French (2000). Contigs built with fingerprints, markers and FPC V4.7. Genome Research 10:1772-1787*.)* into 4599 BACs (74.8%) resulted in 234 contigs with 2-125 BACs per contig. Validation on the published genome sequence by BLAST analysis of the sequence reads showed that approximately 52,000 reads gave 100% hits, covering 99% of the genome with a maximum gap of 125 Kbp. There were 50.000 unique hits; on average 2,355 bp between tags and 80% of all EcoRI sites were represented.

### Melon

Melon has an estimated 450Mbp genome size.

47,616 BACs derived from EcoRI and HindIII libraries, totalling about 13 genome equivalents were analysed. 50% of all reads were deconvolutable to BACs (40,063 BACs; 85%) and were uniquely tagged. Available for contig building: 9,417,2459 Illumina GA II reads of 36 bases; obtained in 5 GA II sequencing runs. 196,256 unique sequence reads were linked to 40,063 BAC clones with on average 33 reads anchored. These reads were assembled into 670 contigs and 8,213 singleton BACs. On average 15 BACs per contig (> 1.8 Mbp) and >90% estimated genome coverage. See fig 3 for contig size distribution.

### Melon:

The melon WGS scaffolds were integrated with melon WGP BAC contigs. The estimated genome size of melon is 450 Mbp.

### Input:

| Data type | # scaffolds / contigs | N50 scaffold size (kb) | Largest scaffold size (Mbp) | Total coverage (Mbp) |
|---|---|---|---|---|
| WGP | 1,88 | 546 | 3,1 | 375 |
| WGS * | 21,126 (> 1000 bp) | 422 | 3,07 | 375 |

| | | | | |
|---|---|---|---|---|
| * comprises: | | | | |

The following sequence data were produced on the GS FLX Titanium platform from nuclear DNA of the melon line:
1) 17 random shotgun runs, comprising a total of 16,171,153 reads
2) 5 3-Kb paired-end runs comprising a total of 4,844,561 reads
3) 3.5 (~20 Kb) long-jump paired end runs, comprising a total of 3,448,598 reads.
4) 1 EcoRI-random end run, comprising a total of 789,048 reads.

The total number of runs performed was 26,5 and the total number of reads generated equals 25,253,360. These reads represent a total of 8,691,334,029 bases (8,69 Gbp) of the melon nuclear genome (i.e. excluding chloroplast and mitochondrial sequences and linker sequences of the paired-end libraries). At an estimated genome size of 450 Mbp, this represents 19,43 fold coverage of the melon genome (breakdown: ~12,44 X random shotgun; ~3,72 X 3 kb PE; ~2,65 X long jump; and ~0,61 X *Eco*RI-random end).

The method connects WGS scaffolds to WGP contigs. As a step in the process, it is determined whether a WGS scaffold overlaps/matches with a single WGP contig or with multiple WGP contigs (based on the presence of WGP tag sequences in the WGS scaffold). Criteria for linking WGS scaffolds to WGP contigs are the number of WGP tags which have a 100% matching sequence. All matches that are made are annotated to know whether they are based on at least 1, 2, or more than 2 matching WGP tag sequences. For WGS scaffolds which cover an entire WGP contig, four different situations have been distinguished, which reflect 4 different confidence levels for linking these WGS and WGP contigs. 5630 WGS scaffolds covering 77 Mbp were linked to 838 single BAC contigs. 470 WGS scaffolds covering 231 Mbp were linked to 903 multiple BAC contigs. These two datasets overlap because the total number of BAC contigs available was 1088, which is below 838+903. Singleton BACs (not placed in BAC contigs) were not included in the analysis.

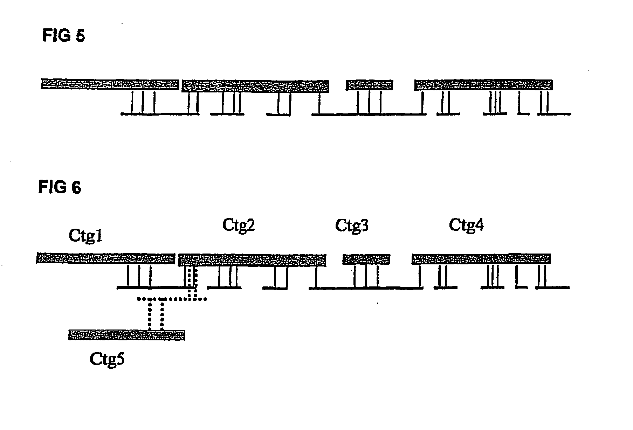

A random BAC contig is taken a "seed" to build a superscaffold based on the presence of shared WGP tag sequences in BAC contigs and their linked WGS scaffolds. See Figure 5 below. In essence, WGP contigs and WGS scaffolds were linked if at least one WGP tag sequence was shared between them and no conflicting tags were identified. The seed will grow until no further links can be made or in case a branchpoint occurs, e.g. when multiple overlapping WGS scaffolds are linked to the same BAC contig (Figure 6).

Following the procedure described above 329 superscaffolds comprising 289 Mbp of the melon genome sequence were generated.

## Claims

1. Method for the determination of a genome sequence comprising the steps of:
- providing a physical map of a sample genome by sequencing fragment ends of fragments of pooled artificial chromosome clones;
- providing a set of sequence reads from the sample genome;
- generating a contig of the physical map and the sequence reads to build a genome sequence.

2. Method for the determination of a genome sequence comprising the steps of:
(a) providing a sample DNA;
(b) generating an artificial chromosome (e.g. BAC, YAC) clone bank wherein each artificial chromosome clone contains part of the sample DNA;
(c) combining the artificial chromosome clones in a pluralityof pools, wherein each clone is present in more than one pool;
(d) providing a set of fragments for each pool;
(e) ligating adaptors to one or both sides of the fragments,
(f) determining the sequence of at least part of the adaptor and part of the fragment;
(g) assigning the fragment sequences to the corresponding clones ;
(h) building a clone-contig thereby generating a physical map of the sample genome;
(i) generating sequence reads from a sample DNA;
(j) aligning the sequence reads and/or contigs or scaffolds from the sequence reads to the clone contig to thereby build a genome sequence/super scaffold.

3. Method according to claim 2, wherein at least one adaptor contains a pool- specific identifier or a degenerate identifier section, respectively, to provide identifier-containing adaptor-ligated fragments.

4. Method according to claims 2 or3, wherein the adapter-ligated fragments are amplified using
- a primer that amplifies at least the identifier and part of the fragment; or
- a primer that contains a section that is complementary to the degenerate section in the adapter and introduces an identifier in the amplified fragment; or
- a primer that is complementary to at least part of the adapter and provides an identifier in the amplified adapter-ligated fragment.

5. Method according to claims 2-4, wherein the fragments for a pool are generated by randomly fragmenting the pools and/or by restriction enzyme fragmentation of the pools.

6. Method according to claims 2-5, wherein the sequence reads are obtained from fragmented sample DNA and/or from one or more artificial chromosome clone(s) of the sample DNA.

7. Method according to claims 2-6, wherein the sequence reads are obtained from randomly fragmented sample DNA and/or from one or more artificial chromosome clone(s) of the sample DNA.

8. Method according to claims 2-6, wherein the sequence reads are obtained from restriction fragments that have been obtained by restriction enzyme fragmentation of the sample DNA and/or from one or more artificial chromosome clone(s) of the sample DNA.

9. Method according to claim 8, wherein the restriction fragments are adapter- ligated restriction fragments.

10. Method according to claim 9, wherein the adapter-ligated restriction fragments are selectively or non-selectively amplified.

11. Method according to any of the previous claims, wherein the sequencing is carried out by means of high-throughput sequencing.

12. Method according to claim 11, wherein the high-throughput sequencing is performed on a solid support.

13. Method according to claim 11 or 12, wherein the high-throughput sequencing is based on Sequencing-by-Synthesis.

14. Method according to claim 11 or 12, wherein the sequencing is based on pyrosequencing.

## Patentansprüche

1. Verfahren zur Bestimmung einer Genomsequenz, umfassend die Schritte:
- Bereitstellen einer physikalischen Karte eines Proben-Genoms durch Sequenzieren der Fragmentenden von Fragmenten gepoolter künstlicher Chromosomenklone;
- Bereitstellen eines Satzes abgelesener Sequenzabschnitte von dem Proben-Genom;
- Erzeugen eines Contigs der physikalischen Karte und der abgelesenen Sequenzabschnitte, um eine Genomsequenz zu erstellen.

2. Verfahren zur Bestimmung einer Genomsequenz, umfassend die Schritte:
(a) Bereitstellen einer Proben-DNA;
(b) Erzeugen einer Bank künstlicher Chromosomenklone (z.B. BAC, YAC), wobei jeder künstliche Chromosomenklon einen Teil der Proben-DNA enthält;
(c) Kombinieren der künstlichen Chromosomenklone in eine Vielzahl von Pools, wobei jeder Klon in mehr als einem Pool vorhanden ist;
(d) Bereitstellen eines Satzes von Fragmenten für jeden Pool;
(e) Ligieren von Adaptern an eine oder beide Seiten der Fragmente;
(f) Bestimmen der Sequenz von mindestens einem Teil des Adapters und einem Teil des Fragments;
(g) Zuweisen der Fragmentsequenzen zu den entsprechenden Klonen;
(h) Erstellen eines Klon-Contigs, wodurch eine physikalische Karte des Proben-Genoms erzeugt wird;
(i) Erzeugen von abgelesenen Sequenzabschnitten aus einer Proben-DNA;
(j) Abgleichen der abgelesenen Sequenzabschnitte und/oder der Contigs oder Gerüste aus den abgelesenen Sequenzabschnitten mit dem Klon-Contig, um dadurch eine Genomsequenz/ein Supergerüst zu erstellen.

3. Verfahren gemäß Anspruch 2, wobei mindestens ein Adapter eine Pool-spezifische Kennung beziehungsweise einen degenerierten Kennungsabschnitt enthält, um kennungshaltige, Adapter-ligierte Fragmente bereitzustellen.

4. Verfahren gemäß den Ansprüchen 2 oder 3, wobei die Adapter-ligierten Fragmente amplifiziert werden unter Verwendung
- eines Primers, der mindestens die Kennung und einen Teil des Fragments amplifiziert, oder
- eines Primers, der einen Abschnitt enthält, welcher zu dem degenerierten Abschnitt in dem Adapter komplementär ist, und eine Kennung in das amplifizierte Fragment einführt, oder
- eines Primers, der zu mindestens einem Teil des Adapters komplementär ist und eine Kennung in dem amplifizierten Adapter-ligierten Fragment bereitstellt.

5. Verfahren gemäß den Ansprüchen 2 - 4, wobei die Fragmente für einen Pool durch zufälliges Fragmentieren der Pools und/oder durch Restriktionsenzymfragmentierung der Pools erzeugt werden.

6. Verfahren gemäß den Ansprüchen 2 - 5, wobei die abgelesenen Sequenzabschnitte von fragmentierter Proben-DNA und/oder von einem oder mehreren künstlichen Chromosomenklonen der Proben-DNA erhalten werden.

7. Verfahren gemäß den Ansprüchen 2 - 6, wobei die abgelesenen Sequenzabschnitte von zufällig fragmentierter Proben-DNA und/oder von einem oder mehreren künstlichen Chromosomenklonen der Proben-DNA erhalten werden.

8. Verfahren gemäß den Ansprüchen 2 - 6, wobei die abgelesenen Sequenzabschnitte von Restriktionsfragmenten, die durch Restriktionsenzymfragmentierung der Proben-DNA erhalten wurden, und/oder von einem oder mehreren künstlichen Chromosomenklonen der Proben-DNA erhalten werden.

9. Verfahren gemäß Anspruch 8, wobei es sich bei den Restriktionsfragmenten um Adapter-ligierte Restriktionsfragmente handelt.

10. Verfahren gemäß Anspruch 9, wobei die Adapter-ligierten Restriktionsfragmente selektiv oder nicht-selektiv amplifiziert sind.

11. Verfahren gemäß einem der vorhergehenden Ansprüche, wobei das Sequenzieren durch Hochdurchsatzsequenzieren ausgeführt wird.

12. Verfahren gemäß Anspruch 11, wobei das Hochdurchsatzsequenzieren auf einer festen Unterlage durchgeführt wird.

13. Verfahren gemäß Anspruch 11 oder 12, wobei das Hochdurchsatzsequenzieren auf Sequenzieren-durch-Synthese beruht.

14. Verfahren gemäß Anspruch 11 oder 12, wobei das Sequenzieren auf Pyrosequenzieren beruht.

## Revendications

1. Procédé pour la détermination d'une séquence génomique comprenant les étapes consistant à:
- fournir une carte physique d'un génome échantillon par séquençage des extrémités des fragments des fragments de clones de chromosomes artificiels regroupés;
- fournir un ensemble de lectures de séquence à partir du génome échantillon;
- générer un contig de la carte physique et des lectures de séquence pour construire une séquence génomique.

2. Procédé pour la détermination d'une séquence génomique comprenant les étapes consistant à:
(a) fournir un ADN échantillon;
(b) générer une banque de clones de chromosomes artificiels (par exemple, BAC, YAC), dans laquelle chaque clone de chromosome artificiel contient une partie de l'ADN échantillon;
(c) combiner les clones de chromosomes artificiels dans une pluralité de groupes, dans lesquels chaque clone est présent dans plus d'un groupe;
(d) fournir un ensemble de fragments pour chaque groupe;
(e) ligaturer des adaptateurs à un ou aux deux côtés des fragments,
(f) déterminer la séquence d'au moins une partie de l'adaptateur et une partie du fragment;
(g) attribuer les séquences des fragments aux clones correspondants;
(h) construire un contig de clones générant ainsi une carte physique du génome échantillon;
(i) générer des lectures de séquence à partir d'un ADN échantillon;
(j) aligner les lectures de séquence et/ou les contigs ou les échafaudages provenant des lectures de séquence au contig de clones pour construire ainsi une séquence génomique/super échafaudage.

3. Procédé selon la revendication 2, dans lequel au moins un adaptateur contient un identificateur spécifique de groupe ou une section d'identificateur dégénérée, respectivement, afin de fournir des fragments ligaturés à un adaptateur contenant un identificateur.

4. Procédé selon les revendications 2 ou 3, dans lequel les fragments ligaturés à un adaptateur sont amplifiés en utilisant
- une amorce qui amplifie au moins l'identificateur et une partie du fragment; ou
- une amorce qui contient une section qui est complémentaire de la section dégénérée dans l'adaptateur et introduit un identificateur dans le fragment amplifié; ou
- une amorce qui est complémentaire d'au moins une partie de l'adaptateur et fournit un identificateur dans le fragment ligaturé à un adaptateur amplifié.

5. Procédé selon les revendications 2 à 4, dans lequel les fragments pour un groupe sont générés par la fragmentation de manière aléatoire des groupes et/ou par la fragmentation par des enzymes de restriction des groupes.

6. Procédé selon les revendications 2 à 5, dans lequel les lectures de séquence sont obtenues à partir d'ADN échantillon fragmenté et/ou à partir d'un ou plusieurs clone(s) de chromosomes artificiels de l'ADN échantillon.

7. Procédé selon les revendications 2 à 6, dans lequel les lectures de séquence sont obtenues à partir d'ADN échantillon fragmenté de manière aléatoire et/ou à partir d'un ou plusieurs clone(s) de chromosomes artificiels de l'ADN échantillon.

8. Procédé selon les revendications 2 à 6, dans lequel les lectures de séquence sont obtenues à partir de fragments de restriction qui ont été obtenus par fragmentation par des enzymes de restriction de l'ADN échantillon et/ou à partir d'un ou plusieurs clone(s) de chromosomes artificiels de l'ADN échantillon.

9. Procédé selon la revendication 8, dans lequel les fragments de restriction sont des fragments de restriction ligaturés à un adaptateur.

10. Procédé selon la revendication 9, dans lequel les fragments de restriction ligaturés à un adaptateur sont amplifiés de manière sélective ou non sélective.

11. Procédé selon l'une quelconque des revendications précédentes, dans lequel le séquençage est réalisé au moyen d'un séquençage à haut débit.

12. Procédé selon la revendication 11, dans lequel le séquençage à haut débit est réalisé sur un support solide.

13. Procédé selon la revendication 11 ou 12, dans lequel le séquençage à haut débit est basé sur un séquençage par synthèse.

14. Procédé selon la revendication 11 ou 12, dans lequel le séquençage est basé sur un pyroséquençage.
